# EUROPEAN PATENT SPECIFICATION

(11) **EP 4 571 311 B1**
(45) Date of publication and mention of the grant of the patent: **27.05.2026**
(21) Application number: 23217300.5
(22) Date of filing: 15.12.2023
(51) Int. Cl.: G01N 33/543

(54) **SWITCHABLE SENSING PLATFORM**
SCHALTBARE MESSPLATTFORM
PLATE-FORME DE DÉTECTION COMMUTABLE

(43) Date of publication of application: 18.06.2025
(73) Proprietor: Imec VZW, 3001 Leuven (BE); Katholieke Universiteit Leuven, 3000 Leuven (BE)
(72) Inventor: ZAPIAIN MERINO, Santino Jesulin, 3000 Leuven (BE); HENRY, Olivier, 1950 Kraainem (BE)
(74) Representative: Winger

(56) References cited:
- EP-A1- 3 779 421
- US-A1- 2010 233 734
- US-B2- 10 866 235

## Description

### Technical field of the invention

The present invention relates to the field of sensing platforms for detecting an analyte.

### Background of the invention

In situ detection of molecular markers is typically performed using immunoassays, such as in a sandwich assay (e.g., ELISA). In typical ELISA assays, a capture antibody (cAb) for selectively binding a particular analyte, e.g., marker (M), is first immobilized onto a substrate (which may, for example, be in plastic or metal). The substrate is exposed to a liquid sample containing the marker, and the immobilized antibody binds or "captures" the marker selectively to form a cAb-M complex. The sample is removed, e.g., flushed away, and the cAb-M complex may be washed to remove any interfering/competing molecules. Subsequently, the cAb-M complex may be detected using electrochemical and optical techniques. However, these techniques often have a rather poor sensitivity (ng-µg/mL).

In a sandwich assay, to enhance sensitivity, a detection antibody (dAb) may be introduced, which may further bind to the cAb-M complex, to form a cAb-M-dAb complex. Therein, the dAb might be tagged with an optical, electrochemical, or enzymatic label (e.g., gold nanoparticles, metallocenes, or horse radish peroxidase, respectively).

Typically, any unbound dAb is flushed away, leaving the cAb-M-dAb complex on the substrate. Subsequently, the cAb-M-dAb complex on the substrate may be detected with very high sensitivity.

In the state of the art, immunoassays are, usually, one-off measurements, wherein continuous measurements are not achieved. Standard immunoassays involve multiple steps, including the addition of various reagents and buffers. This makes them unsuitable for real-time, highly sensitive measurements in an automated manner.

This limitation is particularly challenging in bioreactor settings, where quick and continuous data are crucial. Analysis techniques of the state of the art, such as T cell proliferation, cytokine production, and other types of cell viability assays (such as MTT, MTS, XTT, T cell killing assay), also suffer from this issue. These methods require complex procedures and extended time periods to collect the necessary data for accurate characterization. These techniques often require lengthy time periods and multiple handlings, leading to significant product waste. To address this, current practice frequently involves offline analysis of the cell culture. This entails collecting a sample from the bioreactor and sending it to a specialized facility for analysis. This process can take several days to weeks to complete, depending on the tests required, and only captures the cell culture conditions at the time that the sample was taken.

There is thus still a need in the art for devices and methods that address at least some of the above issues.

US2010/233734 A1 and EP3779421 A1 disclose switchable sensing platforms for detecting an analyte.

US10866235 B2 discloses a sensing platform with ligand being immobilised by click chemistry.

### Summary of the invention

It is an object of the present invention to provide a good switchable sensing platform for detecting an analyte. It is a further object of the present invention to provide a good method for detecting whether an analyte is present in a solution.

The above objective is accomplished by a switchable sensing platform and a method according to the present invention.

In a first aspect, the present invention relates to a switchable sensing platform for detecting an analyte, comprising:
- A transducer comprising a surface,
- a capture molecule, immobilized on said surface, and adapted for bonding with the analyte, and
- a molecular assembly, immobilized on said surface, comprising:
   ∘ a first molecular subunit immobilized on the transducer surface, and
   ∘ a second molecular subunit attached to the first molecular subunit, the second molecular subunit being releasable from the first molecular subunit upon application of a stimulus, e.g., an electrical, optical, or thermal stimulus, to the transducer, and comprising a moiety adapted so that, upon said release of the second molecular subunit, the ability of the switchable biosensing platform to detect the analyte is enhanced.

In a second aspect, the present invention relates to a method for detecting whether an analyte is present in a solution, the method comprising:
a) providing the switchable sensing platform according to embodiments of the first aspect of the present invention,
b) exposing the switchable sensing platform to the solution,
c) applying a stimulus to the transducer (e.g., electrical, optical, or thermal) so as to release the second molecular subunit from the first molecular subunit, and then
d) determining whether the analyte is bonded to the capture molecule.

It is an advantage of embodiments of the present invention that switching of the sensing platform may be straightforward. It is an advantage of embodiments of the present invention that switching of the sensing platform may be performed without changing the (chemical) environment, such as pH or ionic activity, near the sensing platform.

It is an advantage of embodiments of the present invention that the switchable sensitivity of the sensing platform enables in situ detection on-demand.

It is an advantage of embodiments of the present invention that a good sensitivity for detecting the analyte may be achieved.

It is an advantage of embodiments of the present invention that the switchable biosensing platform may be compact enough for in-line use within a disposable cell culture media reactor, as commonly used in current cell manufacturing processes.

It is an advantage of embodiments of the present invention that no additional reagents may be required. It is an advantage of embodiments of the present invention that the switchable biosensing platform may only require applying a potential to the surface to release the second molecular subunit.

It is an advantage of embodiments of the present invention that limited restrictions on the capture molecule exists, thereby allowing for the detection of a wide range of analytes.

It is an advantage of embodiments of the present invention that the second molecular subunit used in the switchable sensing platform may be nontoxic, so that its release upon activation, e.g., application of an electrical, thermal or optical excitation, may be harmless, e.g., towards cell cultures.

It is an advantage of embodiments of the present invention that semi-continuous in-line sensing of analytes may be enabled, such as of biomarkers in cell culture reactors, i.e., bioreactors. It is an advantage of embodiments of the present invention that they do not only facilitate the manufacturing of cell-derived products but also enable real-time monitoring of the cell culture's health. It is an advantage of embodiments of the present invention that the production costs of cell manufacturing can be reduced. This may be achieved by identifying sub-optimal cell cultures more quickly than traditional offline analysis techniques, which can take several days to weeks. It is an advantage of embodiments of the present invention that scalability of the production of cell therapies may be facilitated.

It is an advantage of embodiments of the present invention that traditional cell manufacturing schemes can be improved upon through in-line, real-time process monitoring. This technology allows for individual batch monitoring, enabling better control, optimization, and in-situ evaluation.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG. 1 is a schematic representation of a first example of a switchable sensing platform for detecting an analyte in accordance with embodiments of the present invention.
FIG. 2 is a schematic representation of the first example of a switchable sensing platform for detecting an analyte in accordance with embodiments of the present invention, after application of the stimulus for releasing the second molecular subunit from the first molecular subunit.
FIG. 3 is a schematic representation of a second example of a switchable sensing platform for detecting an analyte in accordance with embodiments of the present invention.
FIG. 4 is a schematic representation of the second example of a switchable sensing platform for detecting an analyte in accordance with embodiments of the present invention, after application of the stimulus for releasing the second molecular subunit from the first molecular subunit.
FIG. 5A is a schematic representation of a third example of a switchable sensing platform for detecting an analyte in accordance with embodiments of the present invention, wherein the stimulus is heat.
FIG. 5B is a schematic representation of the third example of a switchable sensing platform for detecting an analyte in accordance with embodiments of the present invention, after application of the stimulus.
FIG. 6A is a schematic representation of a fourth example of a switchable sensing platform for detecting an analyte in accordance with embodiments of the present invention, wherein the stimulus is electromagnetic radiation.
FIG. 6B is a schematic representation of the fourth example of a switchable sensing platform for detecting an analyte in accordance with embodiments of the present invention, after application of the stimulus.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled" should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a switchable sensing platform for detecting an analyte, comprising:
- a transducer comprising a surface,
- a capture molecule, immobilized on said surface, and adapted for bonding with the analyte, and
- a molecular assembly, immobilized on said surface, comprising:
   ∘ a first molecular subunit immobilized on the transducer surface, and
   ∘ a second molecular subunit attached to the first molecular subunit, the second molecular subunit being releasable from the first molecular subunit upon application of a stimulus to the transducer, and comprising a moiety adapted so that, upon said release of the second molecular subunit, the ability of the switchable biosensing platform to detect the analyte is enhanced.

In embodiments, the transducer may comprise an electrode comprising said surface, and the stimulus may be an electrical current. The electrode may be electrically coupled to a controller comprising a current or voltage source for applying said electrical current.

In embodiments, the transducer may be adapted to be heated and the stimulus may be heat. In embodiments, the transducer may comprise a heating element comprising said surface. In embodiments, the transducer may comprise a thermally conductive substrate comprising the surface, preferably thermally coupled to a heating element, and the stimulus may be heat applied to said thermally conductive substrate, e.g., by said heating element. The thermally conductive substrate may be formed of a metal. The heating element may be electrically coupled to a controller for applying a current or voltage to the heating element for applying the heat.

In embodiments, the transducer may comprise an optical transducer, such as a transparent substrate (e.g., glass, ITO, or ultra-thin metal film) or a waveguide, comprising the surface, and the stimulus may be electromagnetic radiation, i.e., a photon. The optical transducer may be optically coupled to a controller comprising a radiation source for applying said electromagnetic radiation.

The transducer to which the stimulus may be applied, may, in turn, apply the stimulus to the first molecular subunit and/or the second molecular subunit, resulting in said release. The stimulus may be transferred from the transducer, e.g., via the surface, to the first molecular subunit and/or the second molecular subunit, which may result in said release of the second molecular subunit from the first molecular subunit.

In embodiments, the molecular assembly may be selected from host-guest complexes, thermo-cleavable molecular assemblies (e.g., nucleic acid hybridization assemblies), photo-cleavable molecular assemblies, electro-cleavable molecular assemblies, and redox molecular assemblies.

In embodiments, the molecular assembly may be a host-guest complex, wherein one of the first molecular subunit and the second molecular subunit is the host and another of the first molecular subunit and the second molecular subunit is the guest. In embodiments, the host comprises a host moiety adapted for coupling, e.g., non-covalently, with a guest moiety of the guest. Typically, the guest is non-covalently bonded to the host, which may permit straightforward breaking of the bond by the application of the stimulus, such as by the electrical current. In preferred embodiments, the host, or host moiety, comprises a cyclodextrin moiety, e.g., an α-, β-, or γ-cyclodextrin moiety, most preferably a β-cyclodextrin moiety. In these embodiments, ferrocene, an anthraquinone or adamantane radical may be the guest moiety. The binding strength of anthraquinone and adamantane to cyclodextrin depends on the oxidation state of anthraquinone and adamantane. Thus, by reduction or oxidation that may be induced by application of the stimulus, e.g., the electrical current, said bond may be weakened or destroyed, resulting in said release.

In embodiments, the guest, or guest moiety, may be a sandwich structure. For example, the sandwich structure may be adapted to exhibit a conformational change upon reduction or oxidation of the sandwich structure. Said reduction or oxidation may be induced by application of the electrical current. In some embodiments, said reduction or oxidation may be induced by application of the electromagnetic radiation (e.g., ionization radiation) to the optical transducer. In preferred embodiments, the guest comprises a metallocene moiety. The metallocene moiety may be oxidized or reduced, which may change its affinity towards the host. Preferably, the metallocene is ferrocene. Most preferably, the host comprises the cyclodextrin moiety, e.g., the β-cyclodextrin moiety, and the guest comprises the ferrocene moiety. It is an advantage of these embodiments that the guest and the host may be efficiently separated from each other on application of the stimulus, e.g., electrical current. Indeed, the interactions between the metallocene moiety, preferably the ferrocene moiety, and the cyclodextrin moiety, preferably the β-cyclodextrin moiety, may be very stable, depending on the oxidation state of the metallocene moiety (more particularly, of the oxidation state of the metal of the metallocene moiety), but may be disrupted by changing the oxidation state of the metallocene.

In embodiments, the molecular assembly may be a redox molecular assembly. In the redox molecular assembly, at least one of the first molecular subunit and the second molecular subunit may be reduced or oxidized, resulting in said release of the second molecular subunit from the first molecular subunit. For example, a bond between the first molecular subunit and the second molecular subunit may be broken by said reduction or oxidation. Said reduction or oxidation may be induced by application of the electrical current to the electrode surface. In some embodiments, said reduction or oxidation may be induced by application of the electromagnetic radiation (e.g., ionization radiation) to the optical transducer. For instance, the first molecular subunit may comprise a quinone moiety and the second molecular subunit may comprise an aminooxy-polyethylene glycol moiety. The quinone moiety can be oxidized and reduced in a manner similar to the ferrocene moiety, which may alter its affinity for the aminooxy-polyethylene glycol. Indeed, the aminooxy-group, which may bind non-covalently to the quinone moiety, may be oxidized, by the applied current, to form an alcohol, which has only weak interaction with the quinone moiety. Although, instead, the second molecular subunit may comprise a quinone moiety and the first molecular subunit may comprise an aminooxy-polyethylene glycol moiety, some quinones are cytotoxic so they are preferably immobilized on the surface, i.e., comprised in the first molecules subunit.

In embodiments, the molecular assembly may be a thermo-cleavable molecular assembly. Preferably, the molecular assembly is thermo-cleavable at a temperature below a temperature where the capture molecule degrades. For instance, the molecular assembly is thermo-cleavable at a temperature below 70°C, more preferably below 60°C. For instance, the first molecular subunit may comprise a nucleic acid sequence or nucleic acid sequences, complementary to a nucleic acid sequence or nucleic acid sequences comprised in the second molecular subunit, thereby forming a hybridization molecular assembly (e.g., DNA). The nucleic acid (e.g., DNA) - nucleic acid (e.g., DNA) assembly may be designed to have a melting temperature below a temperature where the capture molecule degrades. Upon addition of the analyte, said heat may be applied to the transducer, or the transducer's temperature may be raised, so as to release the second molecular subunit from the first molecular subunit.

In embodiments, the molecular assembly may be a photo-cleavable molecular assembly. The molecular assembly may be tethered to the optical transducer (e.g., transparent substrate, waveguide) via a photocleavable linker (e.g., benzoin ester, carbazole, cinnamate, coumarin, cyanin, nitrophenylethyl, or nitrobenzyl). The photocleavable linker may bind the first molecular subunit to the second molecular subunit. Upon addition of the analyte, the transducer's surface may be illuminated so as to release the second molecular subunit from the first molecular subunit.In embodiments, the molecular assembly may be an electro-cleavable molecular assembly. The molecular assembly may be tethered to the electrode of the transducer. For example, the first molecular subunit may be connected via an electro-cleavable linker, such as a metal linker, to the second molecular subunit, wherein the electro-cleavable linker may be broken by application of a current. Alternatively, the first molecular subunit may comprise the electro-cleavable linker, wherein the second molecular subunit is released when the electro-cleavable linker is broken. The metal linker may comprise a metal nanoparticle, which may comprise, e.g., copper, nickel or ruthenium. The metal linker may be electrochemically oxidized to release the second molecular subunit. In these embodiments, for forming the molecular assembly, the transducer surface may be patterned with the metal linker, onto which the second molecular subunit may be selectively or preferentially immobilized.

In embodiments, the capture molecule comprises a capture moiety adapted for selectively binding to the analyte. In embodiments, the capture molecule is or comprises a capture antibody. The specific type of capture molecule may depend on the specific type of analyte that is to be detected. Preferably, the capture molecule is selective so that it may bind to the analyte but not to another molecule.

The analyte may be any type of analyte that may bind to the capture molecule. For example, the analyte may be a protein, an antigen, or another biomolecule.

In embodiments, the moiety of the second molecular subunit is adapted for preventing at least partially the bonding of the analyte with the capture molecule when the first and the second molecular subunits are attached to one another, and for enabling the bonding of the analyte with the capture molecule when the first and the second molecular subunits are detached from one another. In embodiments, the moiety, i.e., the moiety of the second molecular subunit, comprises a portion, extending farther from the transducer surface than the capture molecule. The second molecular subunit, when attached to the first molecular subunit, may sterically hinder the analyte from binding with the capture molecule. Said steric hindrance may be absent when the second molecular subunit has been released from the first molecular subunit. It is an advantage of embodiments of the present invention that the chemical environment of the sensing platform may be the same after release of the second molecular subunit. In embodiments, the first molecular subunit may comprise a first chain having a length of up to 1000 atoms, preferably from 10 to 300 atoms, more preferably from 10 to 50 atoms. A short first chain may enable fast and reliable transfer of the stimulus, e.g., photon transfer or heat transfer or electron transfer, between the transducer surface and the moiety binding the first and second molecular subunit to each other, e.g., between the transducer surface and the host or guest moiety. The first chain may, at a first end, be coupled or connected to the transducer surface. The first chain may, at a second end, be coupled to the second molecular subunit, or to the host or guest moiety comprised in the first molecular subunit. In embodiments, the second molecular subunit may comprise a second chain having a length of up to 10000 atoms, preferably from 100 to 8000 atoms, more preferably from 1000 to 7000 atoms. The second chain may, at a first end, be coupled to the first molecular subunit, or to the host or guest moiety comprised in the second molecular subunit. A second end of the second molecular subunit may be a terminal end. In embodiments, the capture molecule comprises a chain having a length of up to 500 atoms, preferably from 10 to 200 atoms. The chain of the capture molecule may, at a first end, be connected to the transducer surface. The chain of the capture molecule may, at a second end, be terminated by the capture moiety. In embodiments, a sum of a number of atoms in the first chain and second chain is larger, e.g., at least 10 %, at least 20% or at least 50% larger, than a number of atoms in the chain of the capture molecule. In embodiments, the number of atoms in the chain of the capture molecule is larger, e.g., at least 10 %, at least 20% or at least 50% larger, than the number of atoms in the chain of the first molecular subunit. In embodiments, a weight, in Dalton, of the sum of the first and second molecular subunit is larger, e.g., at least 10 %, at least 20% or at least 50% larger, than a weight, in Dalton, of the capture molecule. In embodiments, a weight, in Dalton, of the first molecular subunit is smaller, e.g., at least 10 %, at least 20% or at least 50% smaller, than a weight, in Dalton, of the capture molecule. It is an advantage of these embodiments that the portion of the molecular assembly may extend farther from the surface than the capture molecule, and that the capture molecule may extend farther from the surface than the first molecular subunit.

In embodiments, the portion that is extending farther from the transducer surface than the capture molecule is hydrophilic. It is an advantage of these embodiments that a water rich environment may be formed over the sensing platform. The portion being hydrophilic may result in a surface formed of said portion having a contact angle of 90° with water, at a temperature of 25°C and an atmospheric pressure of 1.0 atm, e.g., at a pH of 7.0. Preferably, said portion provides the second molecular subunit with hydrophilicity. The second molecular subunit being hydrophilic means that a surface formed of said second molecular subunit has a contact angle of 90° with water, at a temperature of 25°C and an atmospheric pressure of 1.0 atm, e.g., at a pH of 7.0.

Preferably, the hydrophilic portion provides the second molecular subunit with hydrosolubility, so that it is soluble in water at a temperature of 25°C and an atmospheric pressure of 1.0 atm, e.g., at a pH of 7.0. For example, the second molecular subunit may comprise a hydrosoluble polymer. It is an advantage of these embodiments that the hydrophilic portion may facilitate removal of the second molecular subunit, away from the first molecular subunit, after its release from the first molecular subunit. In embodiments, the portion comprises, or is terminated with, a hydrophilic functional group, which may for instance be selected from: a sulphate, a sulphonate, an amino group, a carbonyl group, a carboxylate, a hydroxyl group, and a phosphate. In embodiments, the portion is a polymer. In embodiments wherein the portion is hydrophilic, the hydrophilic portion may be a polymer. For instance, it can be a polymer selected from a polyether, a peptide, a nucleic acid, and a zwitterionic polymer chain. The polyether may be polyethylene glycol. It is an advantage that the hydrophilic portion, e.g., of the polyethylene glycol, may provide a non-fouling layer that may prevent non-specific interactions.

In embodiments, the moiety, i.e., the moiety of the second molecular subunit, comprises an affinity reagent, such as a detection antibody, adapted for bonding with the analyte when the analyte is bonded to the capture molecule. The affinity reagent may facilitate detection of the analyte, e.g., the affinity reagent comprises a group for generating a signal in a technique for determining whether the analyte is present. The affinity reagent may be a biological affinity reagent, e.g., an antibody or a nanobody. The affinity reagent may be a synthetic affinity reagent, e.g., an aptamer or an imprinted polymer.

In embodiments, the first molecular subunit may comprise a first chain having a length of up to 500 atoms, preferably from 5 to 200 atoms, more preferably from 10 to 50 atoms. The first chain may, at a first end, be coupled or connected to the transducer surface. The first chain may, at a second end, be coupled to the second molecular subunit, or to the host or guest moiety comprised in the first molecular subunit. In embodiments, the second molecular subunit may comprise a second chain having a length of up to 500 atoms, preferably from 10 to 200 atoms. The second chain may, at a first end, be coupled to the first molecular subunit, or to the host or guest moiety comprised in the second molecular subunit. A second end of the second molecular subunit may be a terminal end. In embodiments, the capture molecule comprises a chain having a length of up to 10000 atoms, preferably from 1000 to 6000 atoms. The chain of the capture molecule may, at a first end, be connected to the transducer surface. The chain of the capture molecule may, at a second end, be terminated by the capture moiety. Each of said chains may be the longest chain of connected atoms within the respective molecule. In embodiments, a sum of a number of atoms in the first chain and second chain is smaller, e.g., at least 10 %, at least 20% or at least 50% smaller, than a number of atoms in the chain of the capture molecule. In embodiments, a weight, in Dalton, of the sum of the first and second molecular subunit is smaller, e.g., at least 10 %, at least 20% or at least 50% smaller, than a weight, in Dalton, of the capture molecule. It is an advantage of these embodiments that the portion of the molecular assembly may extend farther from the surface than the capture molecule, and that the capture molecule may extend farther from the surface than the first molecular subunit.

In preferred embodiments, the capture molecule is distinct from the molecular assembly. In embodiments, the capture molecule may comprise or be a capture antibody.

In some embodiments, the capture molecule may comprise the first molecular subunit. In these embodiments, the capture moiety may be bonded to the surface by the first molecular subunit. Typically, the capture moiety is, in these embodiments, not released on application of the stimulus. In embodiments, the capture molecule can be immobilized on said surface by the intermediate of the molecular assembly. In other words, the capture molecule 41 can be immobilized on the molecular assembly which can itself be immobilized on said surface.

In embodiments, a plurality of capture molecules and molecules assemblies are connected to the surface of the transducer.

The transducer may be connected to a controller. The controller may be adapted for applying the stimulus, e.g., the heat, the electromagnetic radiation, or the electrical current, to the transducer. The controller may be adapted for sensing whether the analyte is bonded to the capture molecule. In embodiments wherein the transducer is the electrode or the thermally conductive substrate comprising the metal, the controller may be adapted for performing electrical impedance spectroscopy or square wave voltammetry to determine whether the analyte is bonded to the capture molecule. In embodiments, the controller is adapted for detecting a mass for determining whether the analyte is bonded to the capture molecule. In embodiments wherein the transducer is the optical transducer, the controller may be adapted for optically detecting whether the analyte is bonded to the capture molecule.

In embodiments, the sensing platform comprises a plurality of transducers, wherein, on each transducer surface, at least one capture molecule and at least one molecules assembly are immobilized. The sensing platform may be adapted to consecutively apply pulses to the different transducers. Thereby, the second molecules subunit may be released from the first molecules subunit immobilized on different transducer surfaces at different times, enabling continuous or time-dependent detection of the analyte.

Any features of any embodiment of the first aspect may be independently as correspondingly described for any embodiment of the second aspect of the present invention.

In a second aspect, the present invention relates to a method for detecting whether an analyte is present in a solution, the method comprising:
a) providing the switchable sensing platform according to embodiments of the first aspect of the present invention,
b) exposing the switchable sensing platform to the solution,
c) applying a stimulus to the transducer so as to release the second molecular subunit from the first molecular subunit, and then
d) determining whether the analyte is bonded to the capture molecule.

Step b may be performed by applying the solution to the switchable sensing platform. For example, the switchable sensing platform may be fluidically coupled to a fluidic channel or inlet through which the solution may be introduced. As one example, the fluidic channel or inlet may fluidically couple the switchable biosensor platform to a bioreactor, for analysing solution of the bioreactor.

Preferably, step c is performed after step b. Alternatively, step c may be performed before step b, wherein, in step c, the switchable sensing platform may be exposed to a further solution, not comprising the analyte, and wherein the second molecular subunit released after application of the current is removed together with the further solution after step c, and before step b.

In embodiments, step d comprises obtaining data by a technique selected from an electrochemical, optical, thermal, and mass sensitive technique. In embodiments, step d comprises obtaining data by a technique, e.g., an electrochemical technique, selected from electrical impedance spectroscopy or voltammetry, e.g., square wave voltammetry. These techniques may be performed by a controller connected to the transducer, which may comprise an electrode. It is an advantage of these techniques that a high sensitivity may be achieved. Alternatively, step d may comprise an optical technique, wherein the analyte bonded to the capture molecule may be optically detected, e.g., via the transducer being an optical transducer. Still alternatively, in embodiments comprising the affinity reagent, e.g., detection antibody, an enzyme-labelled affinity reagent or enzyme-labelled antibody may be added to bind to the affinity reagent, e.g., detection antibody, generating a signal (e.g., an electrochemical or optical signal, such as a change in colour) when bonded. In embodiments, the controller is connected to the transducer for determining thermal properties, e.g., a heat capacity, of molecules bonded to the transducer for determining whether the analyte is bonded to the capture molecule.

In embodiments, step d may be adapted for determining an amount of analyte. For example, the switchable biosensing platform may comprise a plurality of capture molecules immobilized on the transducer surface, and the technique performed in step d may generate a signal from the amount of analyte bonded to the capture molecules may be derived. For example, the signal may be proportional to the amount of analyte bonded to the capture molecules.

In embodiments, the method comprises, after step d, a step e of:
e1) removing any analyte from the capture molecule, then
e2) exposing the switchable biosensing platform to a molecule having the same structure as the second molecular subunit, and applying a stimulus, e.g., an electrical current, to the transducer, e.g., to the electrode, so as to attach the molecule to the first molecular subunit, thereby regenerating the molecular assembly.

It is an advantage of these embodiments that the same switchable sensing platform may be used for a plurality of measurements, e.g., for detecting the analyte in a plurality of solutions. Step e1 may depend on the analyte and on the capture molecule, namely on how the analyte may be removed from the capture molecules, e.g., on how the bond between the analyte and the capture molecule may be broken.

In embodiments wherein the capture molecule is an antibody, one of the gentlest methods for removing any analyte from the capture molecule is manipulating the pH. By shifting the pH outside the antibody's optimal binding range, the antigen-antibody interaction is weakened, leading to the release of the antigen. Once the antigen is removed, restoring the pH to physiological levels allows the antibody to regain its binding capability.

Another approach involves using high ionic strength buffers. This method weakens the electrostatic interactions crucial for the antigen-antibody binding. It's generally milder than pH shifts and can effectively release the antigen without compromising the antibody. Following antigen removal, replacing the high ionic strength buffer with a standard physiological one reinstates normal conditions.

Applying a non-ionic detergent, such as Triton X-100, represent an alternative strategy. These non-denaturing detergents can disrupt the antigen-antibody interactions.

Temperature fluctuations, though less commonly used, can sometimes aid in antigen removal. Gentle heating followed by cooling might weaken the antigen-antibody interaction enough to release the antigen.

Lastly, elution buffers with mild chaotropic agents, like low concentrations of urea, can disrupt the antigen-antibody interactions.

In some embodiments, an electrical current may be applied to the electrode so as to break the bond between the analyte and the capture molecule. In embodiments, a temperature change or illumination may be applied so as to break the bond between the analyte and the capture molecule.

A change in signal for said release, e.g., on oxidation or reduction of the metal of the metallocene for release of the second molecular subunit, may be larger than that when the analyte is bonded to the capture molecule. In embodiments, the method comprises detecting a baseline, before step c, and/or after step c and before step d, wherein step d comprises comparing the data with said baseline. It is an advantage of these embodiments that the signal that is due to the analyte bonded to the capture molecule may be isolated from signals that are due to release of the second molecular subunit.

Any features of any embodiment of the second aspect may be independently as correspondingly described for any embodiment of the first aspect of the present invention.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

### Example 1: A switchable sensing platform for detecting an analyte comprising a blocking layer

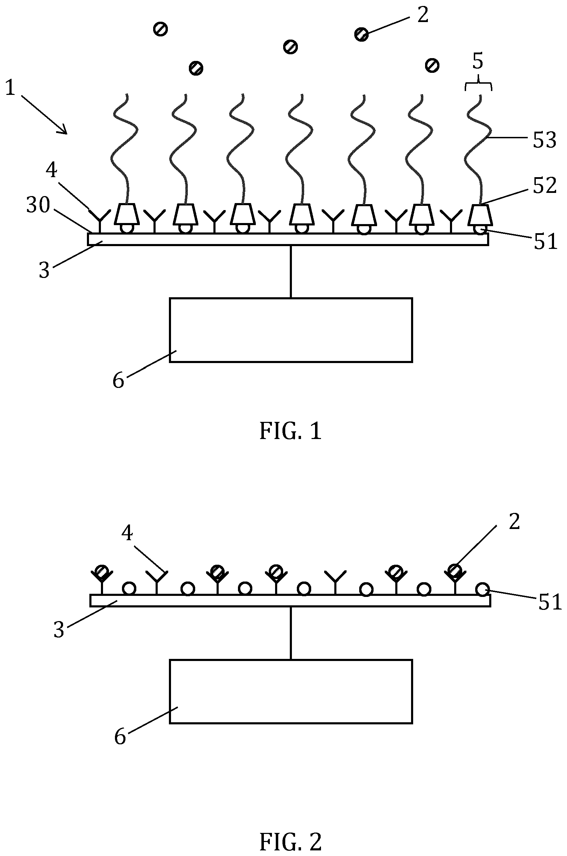

Reference is made to FIG. 1, which is a first example of a switchable sensing platform 1 for detecting an analyte 2, in accordance with embodiments of the present invention.

In this example, the switchable sensing platform 1 comprises a transducer 3 comprising a surface 30. The switchable sensing platform 1 comprises a capture molecule 4, immobilized on said surface 30. For example, the capture molecule 4 may be bonded, e.g., covalently bonded, to the surface 30. The capture molecule 4 is adapted for bonding with the analyte 2.

The switchable sensing platform 1 further comprises a molecular assembly 5 immobilized on the surface 30. The molecular assembly 5 comprises a first molecular subunit 51 immobilized on the transducer surface 30. For example, the first molecular subunit 51 may be bonded, e.g., covalently bonded, to the surface 30. The molecular assembly 5 comprises a second molecular subunit 52 attached to the first molecular subunit 51.

The second molecular subunit 52 comprises a moiety 53 adapted so that, upon said release of the second molecular subunit 52 from the first molecular subunit 51, the ability of the switchable biosensing platform 1 to detect the analyte 2 is enhanced. In particular, in this example, the moiety 53 is adapted for preventing at least partially the bonding of the analyte 2 with the capture molecule 4 when the first molecular subunit 51 and the second molecular subunit 52 are attached to one another and for enabling the bonding of the analyte 2 with the capture molecule 4 when the first molecular subunit 51 and the second molecular subunit 52 are detached from one another.

In this example, the moiety 53 of the second molecular subunit 52 comprises a portion, extending farther from the transducer surface 30 than the capture molecule 4. As said portion of the moiety 53 extends farther from the transducer surface 30 than the capture molecule 4, the analyte 2 may be blocked by said portion of the moiety 53 from approaching the capture molecule 4. Therefore, the sensitivity of the switchable sensing platform 1 towards the analyte 2, when the second molecular subunit 52 is bonded to the first molecules subunit 51, may be low.

The second molecular subunit 52 is releasable from the first molecular subunit 51 upon application of a stimulus to the transducer 3. The transducer 3 may apply the stimulus to the first molecular subunit 51 and/or the second molecular subunit 52, which may result in said release. Said stimulus may be provided by a controller 6. In this example, the molecular assembly 5 is a host-guest complex, wherein the second molecular subunit 52 is the host and the first molecular subunit 51 is the guest. For example, by the application of the stimulus being an electrical current, the guest, i.e., the first molecular subunit 51, may be reduced or oxidized, which may result in a weakened interaction, e.g., breaking of the bond, with the host, i.e., the second molecular subunit 52.

Reference is made to FIG. 2, wherein a stimulus has been applied to the transducer 3 to release the second molecular subunit from the first molecular subunit 51. In this example, the capture molecule 4, or at least part of the capture molecule 4 for binding to the analyte 2, extends farther from the transducer surface 30 than the first molecular subunit 51 and may, hence - after said release of the second molecular subunit from the first molecular subunit 51 - be easily approached by the analyte 2. As a result, the capture molecule 4 may bind to the analyte 2.

To determine whether the analyte 2 is bonded to the capture molecules 4, data may, for example, be obtained by voltammetry, such as square wave voltammetry, when the transducer 3 is an electrode 3. In this technique, a voltage may be applied by the controller 6 to the electrode 3, and a current resulting from said voltage at the electrode 3 may be sensed by the controller 6. When the obtained data, i.e., the current at the electrode 3 as dependent on the voltage at the electrode 3, indicates that analyte 2 is bonded to the capture molecule 4, it may be determined that analyte 2 is present in the solution.

Alternatively, to determine whether the analyte 2 is bonded to the capture molecule 4, data may be obtained by electrical impedance spectroscopy. In this technique, an alternating current may be applied by the controller 6 to the transducer 3 when it is an electrode 3, thereby generating an electric field near the electrode 3, and an impedance at the electrode 3 to the alternating current may be sensed by the controller 6. The data may be obtained by measuring the impedance at a range of frequencies of the alternating current, so that the data comprises the impedance as a function of said frequency. When the obtained data indicates that analyte 2 is bonded to the capture molecule 4, it may be determined that analyte 2 is present in the solution.

However, the technique for determining whether the analyte 2 is bonded to the capture molecule 4 may depend on the type of transducer 3. For example, the transducer 3 may be an optical transducer and the technique may be an optical technique.

After detecting whether the analyte 2 is bonded to the capture molecule 4, i.e., present in the solution, the switchable sensing platform may be regenerated for further use. The analyte 2 bonded to the capture molecule 4 may be removed. This may be achieved, for example, by rinsing with a specific solution for removing the analyte 2 from the capture molecule 4, or by the application of a stimulus, e.g., an electric current or a light beam, by the controller 6 via the transducer 3. Subsequently, the switchable biosensing platform 1 may be exposed to a molecule having the same structure as the second molecular subunit 52. For example, the switchable biosensing platform 1 may be fluidically coupled to a microfluidic device that provides said molecule. When the transducer 3 is the electrode, an electrical current (typically having a sign that is inverted with respect to an electrical current for releasing the second molecular subunit 52 from the first molecular subunit 51) may be applied to the electrode 3 so as to attach the molecule to the first molecular subunit 51. Indeed, in this example, said electrical current may reduce or oxidize the guest 51 (e.g., metallocene) back to the oxidation state in which it efficiently binds to the host 52. Thereby, the molecular assembly 5 may be regenerated, so that the switchable biosensing platform 1 as shown in FIG. 1 may again be obtained.

### Example 2: A switchable sensing platform for detecting an analyte comprising a detection antibody

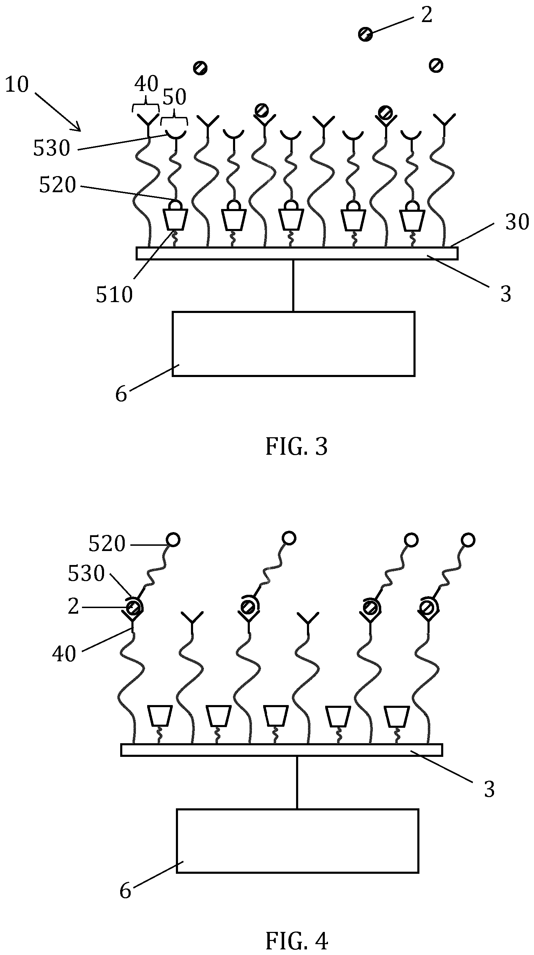

Reference is made to FIG. 3, which is a second example of a switchable sensing platform 10 for detecting an analyte 2, in accordance with embodiments of the present invention.

The switchable sensing platform 10 comprises a transducer 3 comprising a surface 30. The switchable sensing platform 10 comprises a capture molecule 40, immobilized on said surface 30. For example, the capture molecule 40 may be bonded, e.g., covalently bonded, to the surface 30. The capture molecule 40 is adapted for bonding with the analyte 2.

The switchable sensing platform 10 further comprises a molecular assembly 50 immobilized on the surface 30. The molecular assembly 50 comprises a first molecular subunit 510 immobilized on the transducer surface 30. For example, the first molecular subunit 510 may be bonded, e.g., covalently bonded, to the surface 30. The molecular assembly 50 comprises a second molecular subunit 520 attached to the first molecular subunit 510.

The second molecular subunit 520 being releasable from the first molecular subunit 510 upon application of a stimulus to the transducer 3. Said stimulus may be applied by a controller 6.

The second molecular subunit 520 comprises a moiety 530 adapted so that, upon said release of the second molecular subunit 520, the ability of the switchable biosensing platform 10 to detect the analyte 2 is enhanced. In this example, the moiety 530 comprises an affinity reagent, e.g., detection antibody, adapted for bonding with the analyte 2 when the analyte 2 is bonded to the capture molecule 4. The affinity reagent may be adapted so that binding of the analyte 2 to the moiety 530 may be prevented when the analyte 2 is not bonded to the capture molecule 40.

In this example, the capture molecule 40 is available for binding to the analyte 2 in solution when the second molecular subunit 520 is attached to the first molecular subunit 510. The approach, and subsequently said binding, of the capture molecule 40 to the analyte 2 may be unhindered by the second molecular subunit 520. For example, a portion of the capture molecule 40, e.g., the portion for binding to the analyte 2, may extend farther from the surface 30 than the second molecular subunit 520, so that steric hindrance for the analyte 2 approaching the capture molecule 40 by the second molecular subunit 520 may be prevented. For example, the portion of the capture molecule 40 for binding to the analyte 2 may be attached to the surface 30 via a polymer chain.

After a period of time during which the analyte 2 has time to bind to the capture molecule 40, the stimulus may be applied by the controller 6 to the transducer 3 so that the second molecular subunit 520 is released from the first molecular subunit 510. In this example, the molecular assembly 5 is a host-guest complex, wherein the second molecular subunit 520 is the guest and the first molecular subunit 510 is the host. The guest 520 may, for example, comprise a metallocene moiety and the host 510 may comprise a cyclodextrin moiety. For example, by the application of the stimulus being an electrical current, the guest, i.e., the second molecular subunit 520, may be reduced or oxidized, which may result in a weakened interaction, e.g., breaking of the bond, with the host, i.e., the first molecular subunit 510.

Reference is made to FIG. 4. After release of the second molecular subunit 520 from the first molecular subunit 510, the detection antibody of the moiety 530 may bind to the analyte 2 bonded to the capture molecule 40. A technique may, then, be applied to detect the detection antibody of the moiety 530 bonded to the analyte 2, which may be straightforward to detect, e.g., more straightforward than detecting the analyte 2 itself.

For example, a technique for detecting whether the analyte 2 is bonded to the capture molecule 40 may have a higher sensitivity in presence of the detection antibody of the moiety 530. Said technique may, for example, comprise an electrochemical characterization technique such as voltammetry, e.g., square wave voltammetry, or electrical impedance spectroscopy, which may be performed by the controller 6, as described in Example 1 above. For example, the detection antibody of the moiety 530 itself may generate a large signal in said techniques. In this example, the metallocene, e.g., ferrocene, of the guest 520, i.e., of the second molecular subunit 520, may generate a large signal in the electrochemical characterization technique, in particular, in the voltammetry. The advantage of using the metallocene of the guest 520 for detecting whether the analyte 2 is bonded to the capture molecule 40 is that the metallocene is, in this example, present anyway, as it was (before said release) used for binding of the guest 520 to the host 510 that comprises the cyclodextrin moiety. Alternatively, the second molecules subunit 520 may comprise a further moiety generating a large signal in said techniques. Still alternatively, the detection antibody of the moiety 530 may generate an optical signal that may be easily detected in an optical technique. The presence of the analyte 2 may, then, be detected or determined by the detected signal resulting from the detection antibody of the moiety 530.

### Example 3: A switchable sensing platform for detecting an analyte wherein the stimulus is heat

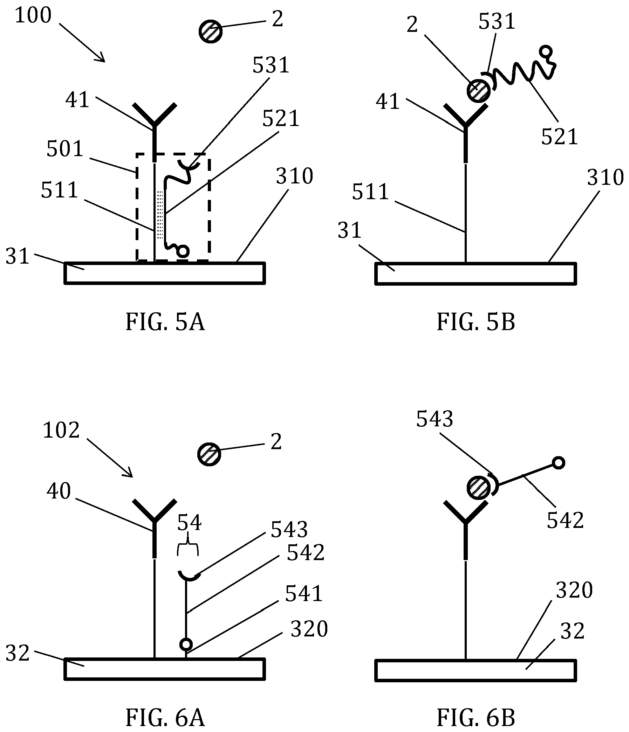

Reference is made to FIG. 5A, which is a schematic representation of a third example of a switchable sensing platform 100 for detecting an analyte in accordance with embodiments of the present invention. In this example, the transducer 31 comprises a heating element, which may provide heat to the surface 310 of the transducer 31.

The switchable sensing platform 100 comprises a capture molecule 41, immobilized on, in this example bonded to, said surface 31, and adapted for bonding with an analyte 2.

The switchable sensing platform 100 further comprises a molecular assembly 501, immobilized on said surface 310.

The molecular assembly 501 comprises a first molecular subunit 511 immobilized on the surface 310. In this example, the first molecular subunit 511 immobilizes the capture molecule 41 on the surface 310 as well.

In some embodiments in accordance with the present invention, the capture molecule 41 comprises the first molecular subunit 511. In embodiments, the capture molecule 41 can be immobilized on said surface 310 by the intermediate of the molecular assembly 501. In other words, the capture molecule 41 can be immobilized on the molecular assembly 501 which can itself be immobilized on said surface 310.

The molecular assembly 501 further comprises a second molecular subunit 521 attached to the first molecular subunit 511, the second molecular subunit 521 being releasable from the first molecular subunit 511 upon application of a stimulus to the transducer 31. The second molecular subunit 521 comprising a moiety 531 adapted so that, upon said release of the second molecular subunit 521, the ability of the switchable biosensing platform 100 to detect the analyte 2 is enhanced.

In the present example, the first molecular subunit 511 and the second molecular subunit 521 comprise complementary nucleotide sequences, which form a double-stranded structure by matching base pairs of the first molecular subunit 511 and the second molecular subunit 521. Said double-stranded structure may become distorted by application of heat, which may break the (typically non-covalent) bonds between the matching base pairs.

Reference is made to FIG. 5B. By applying the heat as stimulus to the transducer 31, the second molecular subunit 521 is released from the first molecular subunit 511. In this example, the capture molecule 41 remains immobilized on the surface 310 via the first molecular subunit 511. The moiety 531 comprises, in this example, an affinity reagent, adapted for bonding with the analyte 2 when the analyte 2 is bonded to the capture molecule 41.

### Example 4: A switchable sensing platform for detecting an analyte wherein the stimulus is electromagnetic radiation

FIG. 6A is a schematic representation of a fourth example of a switchable sensing platform 102 for detecting an analyte 2 in accordance with embodiments of the present invention. The switchable sensing platform 102 comprises a transducer 32 comprising a surface 320.

In this example, the transducer 32 is an optical transducer 32, e.g., a transparent substrate (e.g., glass, ITO, or ultra-thin metal film) or waveguide, and the stimulus is electromagnetic radiation.

The switchable sensing platform 102 comprises a capture molecule 40, immobilized on said surface 320, and adapted for bonding with the analyte 2.

The switchable sensing platform 102 comprises a molecular assembly 54, immobilized on said surface 330.

The molecular assembly 54 comprises a photocleavable linker. The photocleavable linker may bind a first molecular subunit 541 to a second molecular subunit 542. Said photocleavable linker may be contained in the first molecular subunit 541. The first molecular subunit 541 is immobilized on the transducer surface 320.

The second molecular subunit 542 comprises a moiety 543 adapted so that, upon said release of the second molecular subunit 542, the ability of the switchable biosensing platform 102 to detect the analyte 2 is enhanced.

Reference is made to FIG. 6B. The second molecular subunit 542 is released from the first molecular subunit upon application of a stimulus that is, in this example, electromagnetic radiation, or a photon, to the optical transducer 32. Said stimulus may be transferred from the optical transducer 32 to the molecular assembly and cleaves the photocleavable linker, thereby releasing the second molecular subunit 542 from the first molecular subunit. In this example, the first molecular subunit, comprising the photocleavable linker, falls apart on incidence of said radiation so that no trace may remain on the surface 320. For example, the products of the cleavage of said photocleavable linker may disperse into the solution. In a different example, however, at least part of the first molecular subunit, or the complete first molecular subunit, may remain on said surface 320.

The moiety 543 comprises, in this example, an affinity reagent, adapted for bonding with the analyte 2 when the analyte 2 is bonded to the capture molecule 41. Instead of the photo-cleavable linker, as described in the above example, the molecular assembly 54 may comprise an electro-cleavable linker, e.g., a metal linker. In that case, the transducer may comprise an electrode to which the molecular assembly 54 is bonded. The second molecular subunit may then be released from the first molecular subunit by application of an electric current to the transducer. The electric current may, for example, oxidize the metal linker. After said release, the affinity reagent of the second molecular subunit may bind to the analyte when that is bonded to the capture molecule.

Whether the affinity reagent is bonded to the analyte that is in turn bonded to the capture molecule, may be quantified by a change in optical or electrical properties of the transducer.

**It** is to be understood that although preferred embodiments, specific constructions and configurations, as well as materials, have been discussed herein for devices according to the present invention, various changes or modifications in form and detail may be made without departing from the scope of this invention. Steps may be added or deleted to methods described within the scope of the present invention.

## Claims

1. A switchable sensing platform (1, 10) for detecting an analyte (2), comprising:
- a transducer (3, 31, 32) comprising a surface (30, 310, 320),
- a capture molecule (4, 40, 41), immobilized on said surface (30, 310, 320), and adapted for bonding with the analyte (2), and
- a molecular assembly (5, 50, 501, 54), immobilized on said surface (30, 310, 320), comprising:
▪ a first molecular subunit (51, 510, 511, 541) immobilized on the transducer surface (30, 310, 320), and
▪ a second molecular subunit (52, 520, 521, 542) attached to the first molecular subunit (51, 510, 511, 541), the second molecular subunit (52, 520, 521, 542) being releasable from the first molecular subunit (51, 510, 511, 541) upon application of a stimulus to the transducer (3, 310, 320), and comprising a moiety (53, 530, 531, 543) adapted so that, upon said release of the second molecular subunit (52, 520, 521, 542), the ability of the switchable biosensing platform (1, 10) to detect the analyte (2) is enhanced.

2. The switchable sensing platform (1, 10) according to claim 1, wherein the molecular assembly (5, 50, 501, 54) is selected from host-guest complexes (5, 50), thermo-cleavable molecular assemblies (501), photo-cleavable molecular assemblies (54), electro-cleavable molecular assemblies, and redox molecular assemblies (5, 50).

3. The switchable sensing platform (1, 10) according to claim 1 or 2, further comprising a controller (6) for applying said stimulus to the transducer (3, 310, 320).

4. The switchable sensing platform (1, 10) according to any of the previous claims, further comprising a controller (6) adapted for sensing whether the analyte (2) is bonded to the capture molecule (4, 40, 41).

5. The switchable sensing platform (1, 10) according to any of the previous claims, wherein the capture molecule (4, 40, 41) is a capture antibody.

6. The switchable sensing platform (1) according to any of the previous claims, wherein the moiety (53) is adapted for preventing at least partially the bonding of the analyte (2) with the capture molecule (4) when the first (51) and the second molecular subunits (52) are attached to one another and for enabling the bonding of the analyte (2) with the capture molecule (4) when the first (51) and the second molecular subunits (52) are detached from one another.

7. The switchable sensing platform (1) according to claim 6, a weight, in Dalton, of the sum of the first (51) and second molecular subunit (52) is larger than a weight, in Dalton, of the capture molecule (4).

8. The switchable sensing platform (1) according to any of the previous claims, wherein the moiety (53) comprises a portion, extending farther from the transducer surface (30) than the capture molecule (4).

9. The switchable sensing platform (1) according to claim 8, wherein the portion is hydrophilic.

10. The switchable sensing platform (1) according to claim 8 or claim 9, wherein the portion is a polymer.

11. The switchable sensing platform (1) according to claim 10 as depending on claim 9, wherein the hydrophilic portion is a polymer selected from a polyether, a peptide, a nucleic acid, and a zwitterionic polymer chain.

12. The switchable sensing platform (10) according to any one of the preceding claims, wherein the moiety (530) comprises an affinity reagent, adapted for bonding with the analyte (2) when the analyte (2) is bonded to the capture molecule (40).

13. The switchable sensing platform (1, 10) according to any one of the preceding claims, wherein the capture molecule (4, 40) is distinct from the molecular assembly (5, 50, 54).

14. A method for detecting whether an analyte (2) is present in a solution, the method comprising:
a) providing the switchable sensing platform (1, 10) according to any of the previous claims,
b) exposing the switchable sensing platform (1, 10) to the solution,
c) applying a stimulus to the transducer (3, 31, 32) so as to release the second molecular subunit (52, 520, 521, 542) from the first molecular subunit (51, 510, 511, 541), and then
d) determining whether the analyte (2) is bonded to the capture molecule (4, 40, 41).

15. The method of claim 14, wherein step d comprises obtaining data by a technique selected from electrochemical, optical, thermal, and mass sensitive technique.

## Patentansprüche

1. Eine schaltbare Messplattform (1, 10) zum Nachweisen eines Analyten (2), umfassend:
- einen Wandler (3, 31, 32), der eine Oberfläche (30, 310, 320) umfasst,
- ein Fänger-Molekül (4, 40, 41), das auf der Oberfläche (30, 310, 320) immobilisiert ist, und zum Binden mit dem Analyten (2) angepasst ist, und
- eine molekulare Anordnung (5, 50, 501, 54), die auf der Oberfläche (30, 310, 320) immobilisiert ist, umfassend:
- eine erste molekulare Untereinheit (51, 510, 511, 541), die auf der Wandleroberfläche (30, 310, 320) immobilisiert ist, und
- eine zweite molekulare Untereinheit (52, 520, 521, 542), die an der ersten molekularen Untereinheit (51, 510, 511, 541) befestigt ist, wobei die zweite molekulare Untereinheit (52, 520, 521,542) von der ersten molekularen Untereinheit (51, 510, 511, 541) bei Anwenden eines Reizes auf den Wandler (3, 310, 320) lösbar ist, und eine Einheit (53, 530, 531, 543) umfasst, die angepasst ist, sodass sich beim Lösen der zweiten molekularen Untereinheit (52, 520, 521, 542) die Fähigkeit der schaltbaren Biomessplattform (1, 10) zum Nachweisen des Analyten (2) verbessert.

2. Die schaltbare Messplattform (1, 10) nach Anspruch 1, wobei die molekulare Anordnung (5, 50, 501, 54) aus Host-Gast-Komplexen (5, 50), thermisch spaltbaren molekularen Anordnungen (501), durch Licht spaltbare molekulare Anordnungen (54), elektrisch spaltbare molekulare Anordnungen, und molekulare Redox-Anordnungen (5, 50) ausgewählt ist.

3. Die schaltbare Messplattform (1, 10) nach Anspruch 1 oder 2, weiter umfassend eine Steuereinheit (6) zum Anwenden des Reizes auf den Wandler (3, 310, 320).

4. Die schaltbare Messplattform (1, 10) nach einem der vorstehenden Ansprüche, weiter umfassend eine Steuereinheit (6), die zum Messen angepasst ist, ob der Analyt (2) an das Fänger-Molekül (4, 40, 41) gebunden ist.

5. Die schaltbare Messplattform (1, 10) nach einem der vorstehenden Ansprüche, wobei das Fänger-Molekül (4, 40, 41) ein Fänger-Antikörper ist.

6. Die schaltbare Messplattform (1) nach einem der vorstehenden Ansprüche, wobei die Einheit (53) zum mindestens teilweise Verhindern des Bindens des Analyten (2) mit dem Fänger-Molekül (4) angepasst ist, wenn die erste (51) und die zweite molekulare Untereinheit (52) aneinander befestigt sind, und zum Ermöglichen der Bindung des Analyten (2) mit dem Fänger-Molekül (4), wenn die erste (51) und die zweite molekulare Untereinheit (52) voneinander gelöst sind.

7. Die schaltbare Messplattform (1) nach Anspruch 6, wobei ein Gewicht, in Dalton, der Summe der ersten (51) und zweiten molekularen Untereinheit (52) größer ist als ein Gewicht, in Dalton, des Fänger-Moleküls (4).

8. Die schaltbare Messplattform (1) nach einem der vorstehenden Ansprüche, wobei die Einheit (53) einen Abschnitt umfasst, der sich weiter von der Wandleroberfläche (30) erstreckt als das Fänger-Molekül (4).

9. Die schaltbare Messplattform (1) nach Anspruch 8, wobei der Abschnitt hydrophil ist.

10. Die schaltbare Messplattform (1) nach Anspruch 8 oder Anspruch 9, wobei der Abschnitt ein Polymer ist.

11. Die schaltbare Messplattform (1) nach Anspruch 10, wenn von Anspruch 9 abhängig, wobei der hydrophile Abschnitt ein Polymer ausgewählt aus einem Polyether, einem Peptid, einer Nukleinsäure, und einer zwitterionischen Polymerkette ist.

12. Die schaltbare Messplattform (10) nach einem der vorstehenden Ansprüche, wobei die Einheit (530) ein Affinitätsreagens umfasst, das zum Binden mit dem Analyten (2) angepasst ist, wenn der Analyt (2) an das Fänger-Molekül (40) gebunden ist.

13. Die schaltbare Messplattform (1, 10) nach einem der vorstehenden Ansprüche, wobei sich das Fänger-Molekül (4, 40) von der molekularen Anordnung (5, 50, 54) unterscheidet.

14. Ein Verfahren zum Nachweisen, ob ein Analyt (2) in einer Lösung vorhanden ist, wobei das Verfahren umfasst:
a)- Bereitstellen der schaltbaren Messplattform (1, 10) nach einem der vorstehenden Ansprüche,
b)- Aussetzen der Schaltbaren Messplattform (1, 10) der Lösung,
c)- Anwenden eines Reizes auf den Wandler (3, 31, 32) um die zweite molekulare Untereinheit (52, 520, 521, 542) von der ersten molekularen Untereinheit (51, 510, 511, 541) zu lösen, und danach
d)- Bestimmen, ob der Analyt (2) an das Fänger-Molekül (4, 40, 41) gebunden ist.

15. Das Verfahren nach Anspruch 14, wobei Schritt d Erhalten von Daten durch eine Technik umfasst, die aus einer elektrochemischen, optischen, thermischen, und massensensitiven Technik ausgewählt ist.

## Revendications

1. Plateforme de détection commutable (1, 10) pour la détection d'un analyte (2), comprenant :
- un transducteur (3, 31, 32) comprenant une surface (30, 310, 320),
- une molécule de capture (4, 40, 41), immobilisée sur ladite surface (30, 310, 320) et apte à se lier à l'analyte (2), et
- un assemblage moléculaire (5, 50, 501, 54), immobilisé sur ladite surface (30, 310, 320), comprenant :
▪ une première sous-unité moléculaire (51, 510, 511, 541) immobilisée sur la surface du transducteur (30, 310, 320), et
▪ une seconde sous-unité moléculaire (52, 520, 521, 542) fixée à la première sous-unité moléculaire (51, 510, 511, 541), la seconde sous-unité moléculaire (52, 520, 521, 542) pouvant être libérée de la première sous-unité moléculaire (51, 510, 511, 541) lors de l'application d'un stimulus au transducteur (3, 310, 320), et comprenant un fragment (53, 530, 531, 543) adapté de sorte que, lors de ladite libération de la seconde sous-unité moléculaire (52, 520, 521, 542), la capacité de la plateforme de biodétection commutable (1, 10) à détecter l'analyte (2) soit améliorée.

2. Plateforme de détection commutable (1, 10) selon la revendication 1, dans laquelle l'assemblage moléculaire (5, 50, 501, 54) est choisi parmi les complexes hôte-invité (5, 50), les assemblages moléculaires thermoclivables (501), les assemblages moléculaires photoclivables (54), les assemblages moléculaires électroclivables et les assemblages moléculaires redox (5, 50).

3. Plateforme de détection commutable (1, 10) selon la revendication 1 ou 2, comprenant en outre un contrôleur (6) pour appliquer ledit stimulus au transducteur (3, 310, 320).

4. Plateforme de détection commutable (1, 10) selon l'une quelconque des revendications précédentes, comprenant en outre un contrôleur (6) adapté pour détecter si l'analyte (2) est lié à la molécule de capture (4, 40, 41).

5. Plateforme de détection commutable (1, 10) selon l'une quelconque des revendications précédentes, dans laquelle la molécule de capture (4, 40, 41) est un anticorps de capture.

6. Plateforme de détection commutable (1) selon l'une quelconque des revendications précédentes, dans laquelle le fragment (53) est adapté pour empêcher au moins partiellement la liaison de l'analyte (2) à la molécule de capture (4) lorsque la première (51) et la seconde sous-unités moléculaires (52) sont fixées l'une à l'autre, et pour permettre la liaison de l'analyte (2) à la molécule de capture (4) lorsque la première (51) et la seconde sous-unités moléculaires (52) sont détachées l'une de l'autre.

7. Plateforme de détection commutable (1) selon la revendication 6, dans laquelle un poids, en Dalton, de la somme de la première (51) et de la seconde sous-unité moléculaire (52) est supérieur à un poids, en Dalton, de la molécule de capture (4).

8. Plateforme de détection commutable (1) selon l'une quelconque des revendications précédentes, dans laquelle le fragment (53) comprend une portion s'étendant plus loin de la surface du transducteur (30) que la molécule de capture (4).

9. Plateforme de détection commutable (1) selon la revendication 8, dans laquelle la portion est hydrophile.

10. Plateforme de détection commutable (1) selon la revendication 8 ou la revendication 9, dans laquelle la portion est un polymère.

11. Plateforme de détection commutable (1) selon la revendication 10 en ce qu'elle dépend de la revendication 9, dans laquelle la portion hydrophile est un polymère choisi parmi un polyéther, un peptide, un acide nucléique et une chaîne polymère zwitterionique.

12. Plateforme de détection commutable (10) selon l'une quelconque des revendications précédentes, dans laquelle le fragment (530) comprend un réactif d'affinité, adapté pour se lier à l'analyte (2) lorsque l'analyte (2) est lié à la molécule de capture (40).

13. Plateforme de détection commutable (1, 10) selon l'une quelconque des revendications précédentes, dans laquelle la molécule de capture (4, 40) est distincte de l'assemblage moléculaire (5, 50, 54).

14. Procédé pour détecter si un analyte (2) est présent dans une solution, le procédé comprenant :
a) la fourniture de la plateforme de détection commutable (1, 10) selon l'une quelconque des revendications précédentes,
b) l'exposition de la plateforme de détection commutable (1, 10) à la solution,
c) l'application d'un stimulus au transducteur (3, 31, 32) de manière à libérer la seconde sous-unité moléculaire (52, 520, 521, 542) de la première sous-unité moléculaire (51, 510, 511, 541), puis
d) la détermination de la liaison ou non de l'analyte (2) à la molécule de capture (4, 40, 41).

15. Procédé selon la revendication 14, dans lequel l'étape d comprend l'obtention de données par une technique choisie parmi une technique électrochimique, optique, thermique et sensible à la masse.
